# EUROPÄISCHE PATENTSCHRIFT

(11) **EP 2 349 110 B1**
(45) Veröffentlichungstag und Bekanntmachung des Hinweises auf die Patenterteilung: **08.02.2017**
(21) Anmeldenummer: 09737333.6
(22) Anmeldetag: 11.09.2009
(51) Int. Cl.: A61F 2/36, A61F 2/30, A61F 2/00

(54) **MODULARE GELENKPROTHESE**
MODULAR JOINT PROSTHESIS
PROTHÈSE ARTICULAIRE MODULAIRE

(30) Priorität: 26.09.2008 DE 102008049123
(43) Veröffentlichungstag der Anmeldung: 03.08.2011
(73) Patentinhaber: Aristotech Industries GmbH, 14943 Luckenwalde (DE)
(72) Erfinder: ANAPLIOTIS, Emmanuell, 14195 Berlin (DE); KRANZ, Kurt, 14163 Berlin (DE); HILSE, Martin, 12105 Berlin (DE); LOB, Günter, 81377 München (DE)
(74) Vertreter: Hannig, Wolf-Dieter
(86) Internationale Anmeldenummer: PCT/DE2009/001272
(87) Internationale Veröffentlichungsnummer: WO 2010/034284

(56) Entgegenhaltungen:
- EP-B1- 1 004 283
- DE-A1- 3 340 767
- DE-A1- 19 633 865
- DE-A1-102008 045 291
- FR-A1- 2 670 108
- US-A- 5 658 349
- US-B1- 6 786 932

## Beschreibung

Die Erfindung betrifft eine modulare Gelenkprothese mit einem gekrümmt ausgebildeten Kopfteil, auf dessen konischen Zapfen eine aufsteckbare Gelenkkugel befestigt ist, und mit einem in den Knochenmarkkanal des Os longum einsetzbaren Nagel mit einem Nagelhals, wobei zwischen Kopfteil und Nagelhals ein Verbindungsmittel vorgesehen ist, das das Kopfteil verdrehsicher hält.

Die Erfindung betrifft ferner eine modulare Gelenkprothese mit einem schaftartig geformten Halsstück, auf dessen konischen Zapfen eine aufsteckbare Gelenkkugel befestigt ist, und mit in den Knochenmarkkanal des Os longum einsetzbaren Nagel mit einem Nagelhals, wobei zwischen Halsstück und Nagelhals des Nagel ein Verbindungsmittel vorgesehen ist, das das Halsstück in vorbestimmter Winkellage verdrehsicher hält.

Aus der DE 33 40 767 A1 ist ein Bausatz für eine Resektionsprothese mit einem Kopfteil und einem Endteil bekannt, von denen das eine Teile einen konischen Zapfen und das andere Teile eine konische Bohrung aufweist, wobei zwischen diesen beiden Teilen mindestens ein an Zapfen und/oder Bohrung angepasstes Zwischenteil vorgesehen ist. Die beim Zusammensetzen aneinanderstoßenden Flächen der unterschiedlichen Teile sind durch aneinander angepasste Ausformungen gegen Verdrehen sicherbar.

Dieser bekannte Stand der Technik hat den Nachteil, dass die Prothese aus einer Vielzahl von Einzelteilen besteht, die eine nachhaltige Lagerhaltung dieser Teile erforderlich macht. Die Einzelteile müssen im zusammengesetzten Zustand eine der Krümmung des Femur angenäherte Gestaltung besitzen, so dass eine an die beim jeweiligen Patienten vorliegenden Bedingungen entsprechende Krümmung und Winkellage nur durch die Vorratshaltung der entsprechenden Teile erreichbar ist, um die natürliche Funktion des zu ersetzenden Gelenks widerherzustellen.

Aus der EP 1 004 283 B1 ist weiterhin ein orthopädisches Prothesensystem für den Ersatz eines gelenkigen Abschnitts eines Röhrenknochens bekannt, die eine Manschette, einen Schaft, einen Ansatz und ein Verbindungselement umfasst. Das Verbindungselement weist eine Verbindungsstange auf, die mit dem Schaft ausgebildet Diese bekannte Lehre mag zwar für verschieden lange Schäfte und Manschetten modular ausgelegt sein, das Verbindungselement selbst hat jedoch eine solche Modularität nicht.

Die DE 196 33 865 A1 beschreibt eine Endoprothese zum Ersatz knöchernder Bereiche im diaphysären und metaphysären Bereich eines Knochens in Form eines Zwischenkörpers mit Friktionsmitteln zu dessen intramedullären Fixation. Der Zwischenkörper ist in Längsrichtung teilbar ausgebildet, wobei die lösbar miteinander verbundenen Teilelemente unter Einschluss mindestens an ihren Endbereichen vorgesehene Ausnehmungen eine sich axial erstreckende Durchführung als Halterung für ein intramedulläres Fixationsmittel in Form eines Knochennagels bildet.

Die US 6 786 932B1 bezieht sich auf ein Femurendimplantat, das ein Femurendgelekteil, ein mit dem Femurendgelenkteil verbundenes Hohlteil und ein mit dem Hohlteil integral verbundenes rohrförmiges Teil umfasst, welches eine zylindrische Durchführung aufweist, die einen einstellbaren Inndurchmesser zur radialen Kompression des Femurschaftes hat und die den Femurendgelenkteil in einer radialen und winklig festgelegten Lage relativ zum Femurschaft hält.

Die FR 2 670 108 zeigt eine modulare Gelenkprothese gemäss des Oberbegriffs der unabhängigen Ansprüche 1 und 2.

Der grundsätzliche Nachteil all dieser bekannten Prothesen besteht darin, dass ihre Längen und Winkellagen nicht einstellbar sind und dadurch die Modularität erheblich eingeschränkt ist.
Des Weiteren hat dieses bekannte Prothesensystem den Nachteil, dass bei einer späteren Revision erhebliche Probleme bei der Lösung der ausschließlich axial wirkenden Verbindungsmittel auftreten können, weil die axialen Verbindungsmittel sich nur axial lösen und entfernen lassen, was wiederum eine unerwünschte Extension von Weich-, Muskel-und Sehnenteilen mit entsprechender Traumatisierung nach sich ziehen kann. Im Übrigen wirkt auch das axial aufzubringende Anzugs- oder Lösungsmoment auf die Verbindungsmittel, insbesondere auf die Nagelschäfte einem festen Sitz im Knochen entgegen.

### Aufgabenstellung

Bei diesem Stand der Technik liegt der Erfindung die Aufgabe zugrunde, eine modulare Gelenkprothese anzugeben, die die Modularität der Prothese durch eine vergrößerte Längenvariabilität erhöht, eine verbesserte Einstellbarkeit der Winkellage bei gleichzeitiger Reduktion der Teileanzahl ermöglicht und Revisionsoperationen vereinfacht.

Diese Aufgabe wird durch eine modulare Gelenkprothese der eingangs genannten Gattung mit den Merkmalen der Ansprüche 1 oder 2 gelöst.

Vorteilhafte Ausgestaltungen der Gelenkprothese sind den Unteransprüchen entnehmbar.

Die erfindungsgemäße Lösung zeichnet sich dadurch aus, dass eine modulare Gelenkprothese mit einer breiten Anwendungsvielfalt, beispielsweise für den proximalen Femur, bei Resektionen infolge von Metastasen im Knochen, Hüft-Revisionen, Infektionen oder Trauma zur Verfügung gestellt werden kann, welche eine hohe Variationsvielfalt bei geringer Teileanzahl und eine verbesserte Einstellbarkeit der Winkellage besitzt.

Die Übertragung der physiologischen Maximalbelastungen, insbesondere Kräfte und Momente, in den
Die Übertragung der Belastungen gelingt aber auch in vorteilhafter Weise durch ein Halsstück verhältnismäßig kurzer Länge, das an seinem distalen Ende zu einem auf den Nagelhals des Nagels aufsteckbaren helmförmigen Aufnahmekopf ausgeformt ist, der eine Einstecköffnung zum Einführen des Nagelhalses oder eines Verbindungsstücks aufweist, wobei der Aufnahmekopf mit einem in Richtung Längsachse des Kopfteils bis etwa in den Schaft verlaufenden Schlitz versehen ist, dem jeweils senkrecht zur Achse positionierte, zueinander korrespondierende Ausnehmungen zur Aufnahme eines Spannmittels zugeordnet sind, das im gespannten Zustand den Nagelhals oder das Verbindungstück im Aufnahmekopf des Kopfteiles ausschließlich mittels eines an sich bekannten Reibschluss spannbackenartig mit einem gegen axiale Verschiebung und Rotation ausreichendes Anzugsmoment klemmend fixiert.
Dies stellt sicher, dass eine physiologische Krafteinleitung in den Femur bei optimaler Anpassung an die Winkellage der beim Patienten vorliegenden Bedingungen erfolgen kann.

Die Erfindung geht von der Erkenntnis aus, dass ein am Nagelschaft des Nagels spannbackenartig angreifendes manschettenartiges Modul einen linienförmig entlang des Umfangs des Nagelhalses verlaufenden Reibschluss zwischen Nagelhals und Durchführung erzeugt, der auch bei größerer Belastung eine axiale Verschiebung und/oder Rotationsbewegung der an der Verbindung beteiligten Teile sicher ausschließt. Die linienhafte Berührung schließt ein Fretting an den Kontaktstellen dadurch aus, dass Hinund Herbewegungen unterbunden werden. Der linienartig wirkende Reibschluss wird durch eine auf der Innenwandung der Durchführung vorgesehene Profilierung erreicht, wobei der Halsdurchmesser des Nagels auf den Innendurchmesser der Durchführung abgestimmt ist.
zwischen Nagelhals und Durchführung erzeugt, der auch bei größerer Belastung eine axiale Verschiebung und/oder Rotationsbewegung der an der Verbindung beteiligten Teile sicher ausschließt. Die linienhafte Berührung schließt ein Fretting an den Kontaktstellen dadurch aus, dass Hin- und Herbewegungen unterbunden werden. Der linienartig wirkende Reibschluss wird durch eine auf der Innenwandung der Durchführung vorgesehene Profilierung erreicht, wobei der Halsdurchmesser des Nagels auf den Innendurchmesser der Durchführung abgestimmt ist.

Mit den Spannmitteln, die in Längsrichtung des Moduls unmittelbar nebeneinander angeordnet sind, kann der Reibschluss durch ein entsprechend hohes Anzugsmoment gesichert werden. Ein hinreichend großes Anzugsmoment wird beim Einschrauben von Innensechskantschrauben in die mit Innengewinde versehene korrespondierende Ausnehmung mit einem Drehmomentschlüssel erzeugt.

Von besonderen Vorteil ist weiterhin, dass die spannbackenartige Verbindung zwischen dem Schaft des Nagels einerseits und dem manschettenartigen Modul oder dem Aufnahmekopf andererseits auch bei einer Revision problemlos gelöst und die Traumatisierung des Körpergewebes im Bereich des Gelenks sehr gering gehalten werden kann. Dies gelingt insbesondere durch die besonders günstige ventrale Zugänglichkeit der Spannmittel sowohl an dem aus Halbschalen gebildeten manschettenartigen Modul als auch an dem geschlitzten Aufnahmekopf, wodurch sich der operative Eingriff wesentlich vereinfacht.

Durch die Längenvariabilität des Moduls auf unterschiedliche Längen ist die erfindungsgemäße Gelenkprothese an die verschiedenartigen anatomischen Gegebenheiten der einzelnen Patienten gut anpassbar. Dadurch, dass der Aufnahmekopf des Halsstücks auf den Nagelschaft des Nagels aufsteckbar und im Reibschluss gehalten ist, kann auch der Antetorsionswinkel stufenlos eingestellt und die Resektionshöhe möglichst gering gehalten werden.

Erfindungswesentlich ist, dass die Verbindungsmittel selbst modular aufgebaut sind, so dass es möglich wird, Einstellungen einer vorhandenen Hüft-TEP zu übernehmen und/oder Komponenten mit unterschiedlichen CCD-Winkeln zu kombinieren.

Besondere Vorteile sind insbesondere dann erreichbar, wenn Module unterschiedlicher oder gleicher Länge durch einen Modulverbinder miteinander verbunden werden, so dass die Längenvariabilität der erfindungsgemäßen Gelenkprothese deutlich vergrößert werden kann.
Die modulare Längenvielfalt der erfindungsgemäßen Gelenkprothese lässt sich gemäß einer vorteilhaften Weiterbildung der Erfindung dadurch erweitern, dass der Modulverbinder mit dem manschettenartigen Modul kombiniert wird, so die Gelenkprothese modulartig zu unterschiedlichen Längen zusammensetzbar ist. Zum Verbinden des Modulverbinders mit dem Nagel ist für diesen Fall zusätzlich ein manschettenartiges Modul mit mindestens zwei in Achsrichtung des Moduls geteilte, lösbar miteinander verbundene Halbschalen vorgesehen, die miteinander eine sich axial erstreckende Durchführung zum Einführen des Nagelhalses in das eine Ende und des Modulverbinders in das andere Ende der Durchführung ausbilden und die koaxial in Längsrichtung des Moduls angeordnete, jeweils senkrecht zur Achse positionierte, zueinander korrespondierende Ausnehmungen zur Aufnahme eines Spannmittels aufweisen, das im gespannten Zustand den Nagelhals in der Durchführung ausschließlich mittels Reibschluss spannbackenartig mit einem gegen axiale Verschiebung und Rotation ausreichenden Anzugsmoment klemmend fixiert.

In weiterer Ausgestaltung der Erfindung besitzen alle Teile der erfindungsgemäßen Gelenkprothese eine raue Oberfläche, um das Einwachsen der Prothese in chirurgisch behandelten Bereich zu beschleunigen.

In einer weiteren vorteilhaften Weiterbildung der Erfindung ist der Außendurchmesser des Nagelhalses an den Innendurchmesser der Durchführung im Verbindungsmodul oder des Aufnahmekopfes des Halsstücks angepasst, wobei Nägel mit unterschiedliche Längen und Schaftlängen, aber mit gleichem Durchmesser des Nagelhalses vorgesehen sind.

Alle Teile der erfindungsgemäßen Gelenkprothese bestehen aus einem körperverträglichen und körperbeständigen, vorzugsweise metallischen Werkstoff, beispielsweise aus Titan, Tantal, Niob oder deren Legierungen.

Weitere Vorteile und Einzelheiten ergeben sich aus der nachfolgenden Beschreibung unter Bezugnahme auf die beigefügten Zeichnungen.

### Ausführungsbeispiele

Die Erfindung soll nachstehend an zwei Ausführungsbeispielen näher erläutert werden.
Es zeigt
Fig. 1 eine Explosionsdarstellung der erfindungsgemäßen Gelenkprothese mit einem abgebogenen langen Kopfteil,
Fig. 2 eine perspektivische Ansicht eines manschettenartigen Verbindungsmoduls zum Verbinden von Nagelschaft und Kopfteil,
Fig. 3a bis 3e eine Draufsicht auf verschiedene Verbindungsmodule eines Bausatzes mit unterschiedlicher Länge,
Fig. 4 eine perspektivische Ansicht eines Modulverbinders,
Fig. 5 eine perspektivische Ansicht eines Anschlag-oder Stützmoduls,
Fig. 6 eine Explosionsdarstellung einer weiteren Variante der erfindungsgemäßen Gelenkprothese mit einem kurzen Halsstück mit Aufnahmekopf,
Fig. 7a bis 7c perspektivische Ansichten des Aufnahmekopfes nach Fig. 6 und
Fig. 8a bis 8c eine Ansicht der Nägel mit unterschiedlicher Länge.

### Beispiel 1

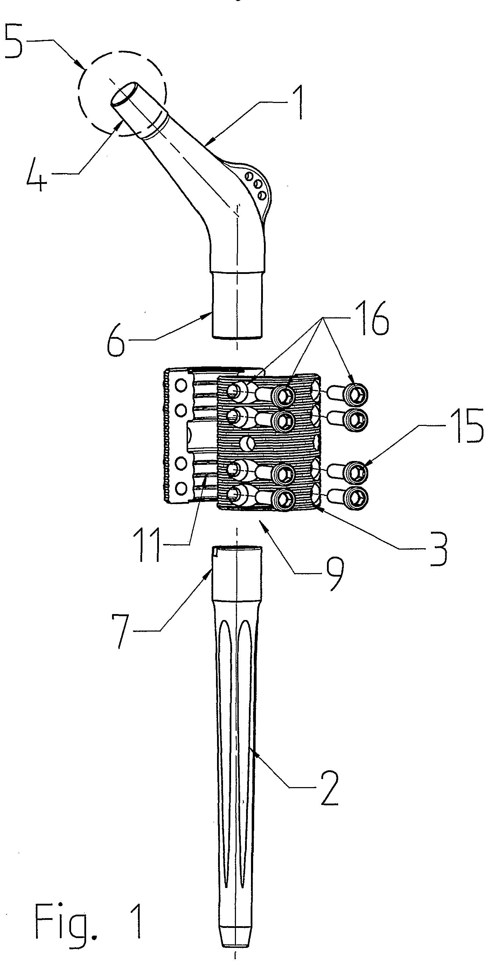

Die Fig. 1 zeigt den grundsätzlichen Aufbau der erfindungsgemäßen Gelenkprothese in einer ersten Ausführungsvariante in Explosionsdarstellung. Die erfindungsgemäße Gelenkprothese, die beispielsweise bei Resektionen aufgrund von Metastasen im Knochen, bei Hüft-Revisionen oder auch bei Infektionen bzw. Trauma eingesetzt wird, setzt sich aus einem entsprechend den biomechanischen Gegebenheiten der Anatomie des jeweiligen Patienten angepassten, gekrümmt ausgebildeten, einstückigen Kopfteil 1, einem Nagel 2, beispielsweise Femurnagel, und einem Verbindungsmodul 3 zusammen.
Auf dem konischen Zapfen 4 des Kopfteils 1 ist eine Gelenkkugel 5 aufsteckbar angeordnet, die in eine nicht dargestellte Gelenkpfanne eingreift. Das distale Ende des Kopfteils 1 ist zu einem zylindrischen Schaft 6 aus Vollmaterial ausgeformt und wird zur Befestigung in das später beschriebene Verbindungsmodul 3 eingeschoben.
Der Nagel 2 wird in den Knochenmarkkanal des nicht dargestellten Os longum eingebracht und verankert. Die Nägel 2 haben unterschiedliche Längen und sind anatomisch an die Kontur des Markkanals entsprechend angepasst. Sie sind durch Verriegelungsschrauben bzw. ein sternförmiges Profil ausreichend gegen Rotation im Markkanal gesichert.

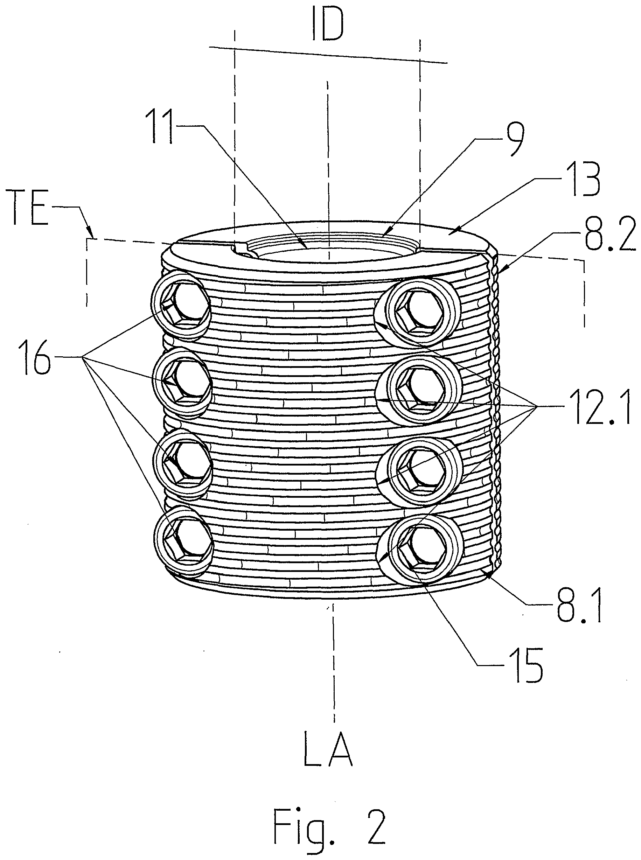

Der Nagel 2 besitzt einen zylindrischen Nagelhals 7 aus Vollmaterial. Mit dem Nagel 2 ist der Schaft 6 des Kopfteils 1 mittels des Verbindungsmoduls 3 starr verbunden, das den Nagelhals 7 und den Schaft 6 jeweils manschettenförmig umfasst. Das Verbindungsmodul 3 ist - wie Fig. 2 zeigt- zweiteilig und besteht aus zwei zylindrischen Halbschalen 8.1 und 8.2, die im Wesentlichen gleichartig ausgebildet sind. Beide Halbschalen 8.1 und 8.2 definieren im zusammengesetzten Zustand eine Durchführung 9, deren innere Wandung 10 eine senkrecht zur Längsachse LA des Moduls angeordnete Profilierung 11 besitzt. Jeweils vier nebeneinander liegende Ausnehmungen 12.1 und 12.2 sind in jede der Halbschalenwandung 13 so eingebracht, dass die Ausnehmungen koaxial zur Längsachse LA des Moduls 3 und senkrecht zu der von den Halbschalen 8.1 und 8.2 virtuell aufgespannten Teilungsebene TE angeordnet sind, so dass beispielsweise zum Verbindungsmodul 3 mindestens 8 Ausnehmungen gehören. In die Ausnehmungen 12.1 der Halbschale 8.2 ist ein Innengewinde 14 eingebracht, in das eine durch die Ausnehmung 12.1 der Halbschale 8.1 eingeführte Innensechskantschraube 15 eingeschraubt werden kann.

Die Halbschalen 8.1 und 8.2 bilden mit der jeweiligen Innensechskantschraube 15 und dem dazu korrespondierenden Innengewinde 14 ein spannbackenartiges Spannmittel 16, das sich beim Anziehen der Innensechskantschraube 15 um den zylinderförmigen Nagelhals 7 legt und zwischen der Profilierung 11 und dem Nagelhals 7 mehrere umfangsmäßig umlaufende linienartige Reibschlussverbindungen erzeugt. Der Außendurchmesser AD des Nagelhalses 7 ist dabei auf den Innendurchmesser ID der Durchführung 9 entsprechend abgestimmt.
Der Widerstand dieser Reibschlussverbindung gegen eine axiale oder eine Rotation der Komponenten lässt sich durch das Aufbringen eines definierten Anzugmomentes auf die Innensechskantschraube 15 sehr genau und exakt einstellen. Durch die Zuordnung von jeweils vier Spannmitteln 16 am Nagelhals 7 wird eine sichere Verbindung zwischen dem Nagel 2 und dem Kopfteil 1 erreicht.
Alle Spannmittel 16 sind nach ventral ausgerichtet und so bei einer späteren Revision problemlos von außen zugänglich, ohne große Bereiche des körpereigenen Gewebes traumatisieren zu müssen.

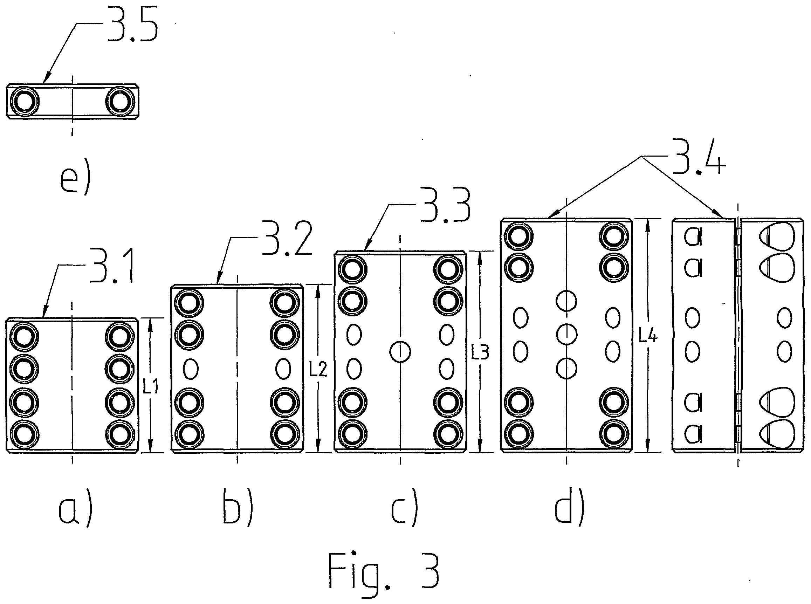

Die Fig. 3a bis 3d zeigen verschiedene Verbindungsmodule 3.1 bis 3.4 mit unterschiedlichen Längen L, beispielsweise ein Verbindungsmodul 3.1 mit einer Länge L1 Fig. 3a), ein Verbindungsmodul 3.2 mit einer Länge L2 (Fig. 3b), ein Verbindungsmodul 3.3 mit einer Länge L3 (Fig. 3c), und ein Verbindungsmodul 3.4 mit einer Länge L4 (Fig. 3d). Es versteht sich, dass auch die Längen der Verbindungsmodule nach den anatomischen Gegebenheiten der jeweiligen Patienten ausgewählt werden.

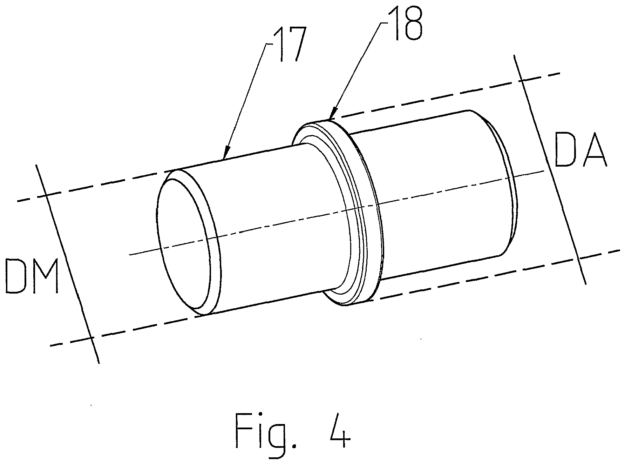

Die Fig. 4 zeigt einen rundstabförmigen Modulverbinder 17, mit dessen Hilfe unterschiedlich lange oder gleichlange Verbindungsmodule 3.1 bis 3.4 aneinanderfügbar sind. Der Modulverbinder 17 besteht aus Vollmaterial, dessen Durchmesser DM auf den Innendurchmesser ID der Durchführung 9 abgestimmt ist. Der Modulverbinder 17 weist mittig einen umlaufenden Anschlag 18 mit einem Durchmesser DA auf, der gegenüber dem Innendurchmesser ID der Durchführung 9 größer ist. Durch Einschieben der jeweiligen Enden des Modulverbinders 17 in das entsprechende Ende der Durchführung 9 der jeweilig zu verbindenden Verbindungsmodule 3.1 bis 3.4 und Spannen der jeweilig zugehörenden Spannmittel 16 wird eine Reibschlussverbindung zwischen dem Modulverbinder 17 und den Verbindungsmodulen hergestellt.

Dadurch ist es möglich, die einzelnen Verbindungsmodule 3.1 bis 3.4 miteinander zu kombinieren und eine große Längenvariabilität zu erreichen.

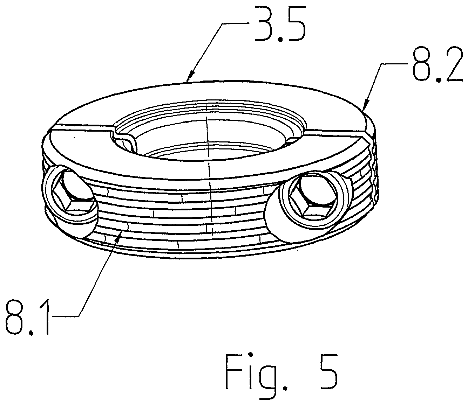

Das in Fig. 3e und 5 gezeigte Anschlag- oder Stützmodul 3.5 dient als eine in der Höhe einstellbare Abstützung gegenüber dem Os longum, beispielsweise Femur.

### Beispiel 2

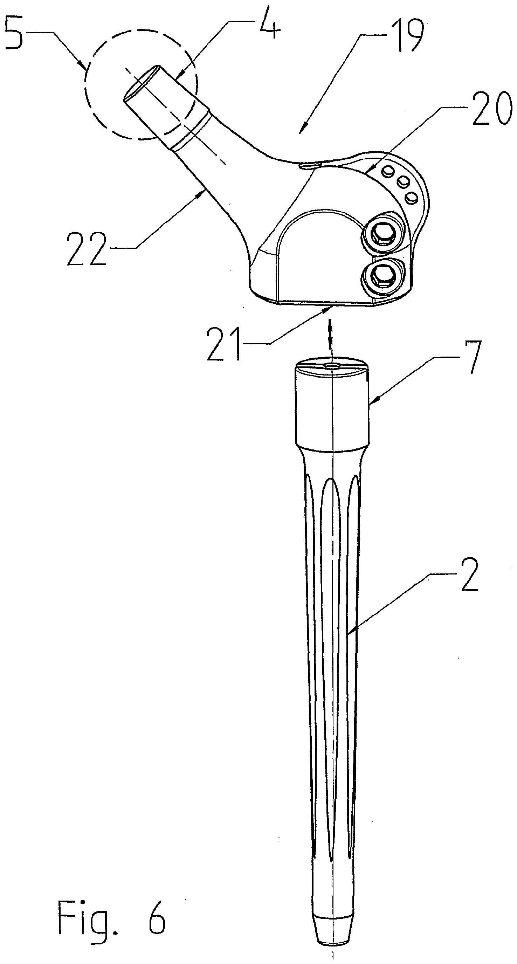

Die Fig. 6 zeigt eine weitere Variante der erfindungsgemäßen Gelenkprothese in Explosionsdarstellung. Diese Gelenkprothese umfasst ein schaftartiges Halsstück 19 verhältnismäßig kurzer Länge und einen Nagel 2, beispielsweise Femurnagel. Das Wesentliche dieser Variante der Erfindung besteht darin, dass das in Beispiel 1 noch separate Verbindungsmodul 3 integraler Bestandteil des schaftartigen Halsstücks 19 wird, so dass nur noch insgesamt zwei Elemente die erfindungsgemäße Gelenkprothese bilden.

Wie im Beispiel 1 auch besitzt das Halsstück 19 einen konischen Zapfen 4, auf dem eine Gelenkkugel 5 aufsteckbar angeordnet ist, die in eine nicht dargestellte Gelenkpfanne eingreift. Das distale Ende des Halsstücks 19 ist zu einem helmartigen Aufnahmekopf 20 ausgeformt, der eine Einstecköffnung 21 zum Einführen des Nagelhalses 7 aufweist. Der Aufnahmekopf 20 ist mit einem in Richtung der Längsachse LA des Halsstücks 19 bis etwa in den Schaft 22 des Halsstücks 19 verlaufenden Schlitz 23 versehen. Dem Schlitz 23 sind in den Seitenwänden 24 jeweils zwei senkrecht zur Längsachse LA positionierte, zueinander korrespondierende Ausnehmungen 25.1 und 25.2 zur Aufnahme eines Spannmittels 26 zugeordnet, so dass sich auch der Antetorsionswinkel stufenlos einstellen lässt.

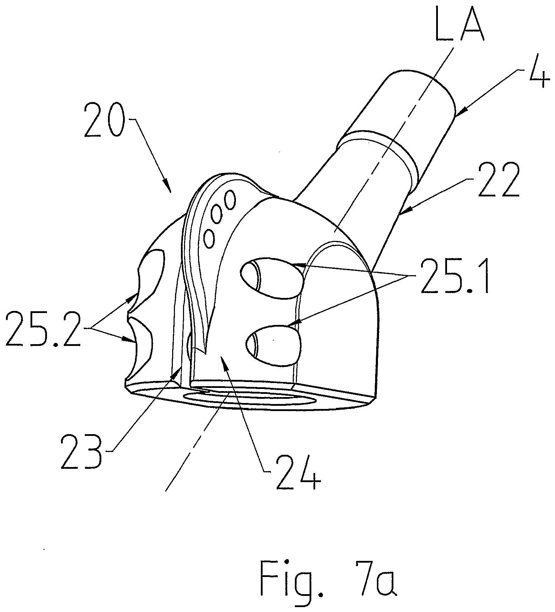

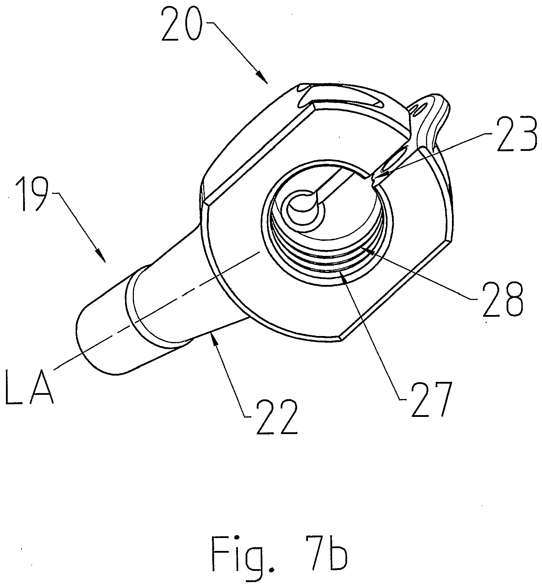

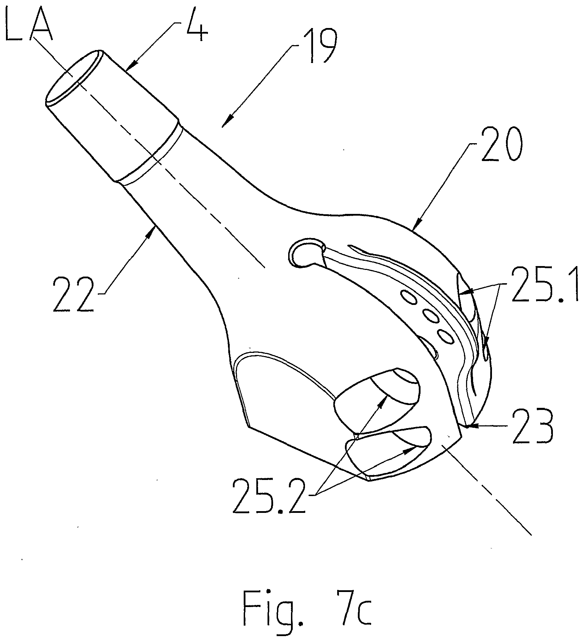

Die innere Wandung 27 des Aufnahmekopfes 20 ist mit einer senkrecht zur Längsachse LA des Halsstücks 19 angeordnete Profilierung 28 versehen (siehe Fig. 7a und 7b).

In die Ausnehmungen 25.1 und 25.2 der Seitenwände 24 ist ein Innengewinde 29 eingebracht, in das eine durch die Ausnehmung 25.1 eingeführte Innensechskantschraube 30 eingeschraubt werden kann.

Die Ausnehmungen 25.1 und 25.2 mit ihrem Innengewinde 28 bilden mit der jeweiligen Innensechskantschraube 30 und dem Schlitz 23 das spannbackenartige Spannmittel 26, das sich beim Anziehen der Innensechskantschraube 30 um den zylinderförmigen Nagelhals 7 legt und zwischen der Profilierung 28 und dem Nagelhals 7 eine umfangsmäßig umlaufende linienartige Reibschlussverbindung erzeugt. Der Außendurchmesser AD des Nagelhalses 7 ist dabei auf den Innendurchmesser ID der Einstecköffnung 21 entsprechend abgestimmt.
Der Widerstand dieser Reibschlussverbindung gegen eine axiale oder eine Rotation der Komponenten lässt sich durch das Aufbringen eines definierten Anzugmomentes auf die Innensechskantschraube 30 sehr genau und exakt einstellen. Durch die Zuordnung von jeweils zwei Spannmitteln 26 am Nagelhals 7 wird eine sichere Verbindung zwischen dem Nagel 2 und dem Halsstück 19 erreicht.
Alle Spannmitteln 26 sind nach ventral ausgerichtet und so bei einer späteren Revision problemlos von außen zugänglich, ohne große Bereiche des körpereigenen Gewebes traumatisieren zu müssen.

Es gehört natürlich auch zur erfindungsgemäßen Lösung, wenn der in Fig. 4 dargestellte Modulverbinder 17, dessen Außendurchmesser AD auf den Innendurchmesser der Einstecköffnung 21 abgestimmt ist, in die Einstecköffnung 21 des Aufnahmekopfes 20 geschoben wird und eine erweiterte Längenvariabilität der Gelenkprothese dadurch erreicht wird, dass der Nagelhals 7 des Nagel 2 und der Modulverbinder 17 durch verschieden lange Verbindungsmodule 3 (siehe Fig. 3a-d) verbunden werden.

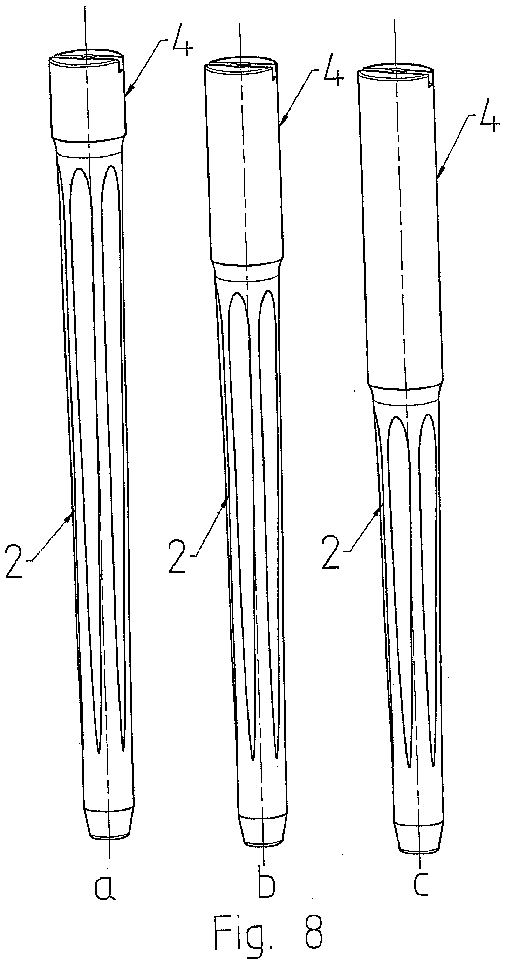

Die Fig. 8a bis c zeigen Beispiele von Nägeln mit unterschiedlicher Länge, deren Halsdurchmesser AD auf den Innendurchmesser ID der Durchführung 7 der jeweiligen Verbindungsmodule 3.1 bis 3.4 bzw. der Einstecköffnung 21 im Aufnahmekopf 20 abgestimmt sind.

Alle Teile der erfindungsgemäßen Gelenkprothese bestehen aus einem körperverträglichen und körperbeständigen metallischen Werkstoff. Die äußeren Oberflächen sind mit einer definierten Rauheit, beispielweise zwischen 20 µm und 80 µm versehen. Dies soll einerseits bei den Reibschlusspartnern den Reibkoeffizienten erhöhen und andererseits das Einwachsen des Implantats fördern.

Die Erfindung beschränkt sich in ihrer Ausführung nicht auf die vorgenannten Ausführungsbeispiele. Vielmehr sind Varianten denkbar, welche von der dargestellten Lösung auch bei grundsätzlich anders gearteten Ausführungen abweichen können.

**Bezugszeichenliste**

| | |
|---|---|
| Kopfteil | 1 |
| Nagel | 2 |
| Verbindungsmodul | 3, 3.1-3.4 |
| Stütz- oder Anschlagmodul | 3.5 |
| Zapfen von 1 | 4 |
| Gelenkkugel | 5 |
| Zylindrischer Schaft von 1 | 6 |
| Nagelhals von 2 | 7 |
| Halbschalen von 3 | 8.1, 8.2 |
| Durchführung in 3 | 9 |
| Innere Wandung von 3 | 10 |
| Profilierung | 11 |
| Ausnehmungen | 12.1, 12.2 |
| Halbschalenwandung | 13 |
| Innengewinde | 14 |
| Innensechskantschraube | 15 |
| Spannmittel | 16 |
| Modulverbinder | 17 |
| Anschlag von 17 | 18 |
| Schaftartiges Halsstück | 19 |
| Aufnahmekopf | 20 |
| Einstecköffnung in 20 | 21 |
| Schaft von 19 | 22 |
| Schlitz | 23 |
| Seitenwände von 19 | 24 |
| Ausnehmung | 25.1, 25.2 |
| Spannmittel | 26 |
| Innere Wandung von 19 | 27 |
| Profilierung | 28 |
| Innengewinde | 29 |
| Innensechskantschraube | 30 |
| Außendurchmesser von 18 | DA |
| Durchmesser von 17 | DM |
| Innendurchmesser von 9 | ID |
| Länge von 3, 3.1-3.4 | L |
| Längsachse von 3, 3.1-3.4 | LA |
| Teilungsebene von 3 | TE |

## Patentansprüche

1. Modulare Gelenkprothese mit einem gekrümmt ausgebildeten Kopfteil, auf dessen konischen Zapfen eine aufsteckbare Gelenkkugel befestigt ist, und mit einem in den Knochenmarkkanal des Os longum einsetzbaren Nagel (2) mit einem Nagelhals (7), wobei zwischen Kopfteil (1) und Nagelhals (7) ein Verbindungsmittel vorgesehen ist, das das Kopfteil verdrehsicher hält, **dadurch gekennzeichnet, dass** das Verbindungsmittel mindestens ein in Achsrichtung des Nagels gelegenes manschettenartiges Modul (3, 3.1-3.4) mit mindestens zwei in Achsrichtung (LA) des Moduls (3, 3.1-3.4) geteilte, lösbar miteinander verbundene Halbschalen (8.1,8.2) umfasst, die miteinander eine sich axial erstreckende Durchführung (9) zum Einführen des Nagelhalses (7) in die Durchführung (9) ausbilden und die koaxial in Längsrichtung des Moduls (3) angeordnete, jeweils senkrecht zur Achse (LA) positionierte, zueinander korrespondierende Ausnehmungen (12.1,12.2) zur Aufnahme eines Spannmittels (16) aufweisen, das im gespannten Zustand den Nagelhals (7) in der Durchführung (9) ausschließlich mittels Reibschluss spannbackenartig mit einem gegen axiale Verschiebung und Rotation ausreichenden Anzugsmoment fixiert.

2. Modulare Gelenkprothese mit einem schaftartig geformten Halsstück, auf dessen konischen Zapfen eine aufsteckbare Gelenkkugel befestigt ist, und mit in den Knochenmarkkanal des Os longum einsetzbaren Nagel (2) mit einem Nagelhals (7), wobei zwischen Halsstück (19) und Nagelhals (7) des Nagels ein Verbindungsmittel vorgesehen ist, das das Halsstück (19) in vorbestimmter Winkellage verdrehsicher hält, **dadurch gekennzeichnet, dass** das Halsstück (19) an seinem distalen Ende zu einem auf den Nagelhals (7) des Nagels aufsteckbaren helmförmigen Aufnahmekopf (20) ausgeformt ist, der eine Einstecköffnung (21) zum Einführen des Nagelhalses (7) oder eines Modulverbinder (17) aufweist, wobei der Kopf (20) mit einem in Richtung Längsachse (LA) des Halsstücks (19) bis etwa in den Schaft (22) verlaufenden Schlitz (23) versehen ist, dem jeweils senkrecht zur Achse (LA) positionierte, zueinander korrespondierende Ausnehmungen (25.1, 25.2) zur Aufnahme eines Spannmittels (26) zugeordnet sind, das im gespannten Zustand den Nagelhals (7) oder Modulverbinder (17) im Aufnahmekopf (20) des Halsstücks (19) ausschließlich mittels an sich bekanntem Reibschluss spannbackenartig mit einem gegen axiale Verschiebung und Rotation ausreichendes Anzugsmoment klemmend fixiert.

3. Gelenkprothese nach Anspruch 2, **dadurch gekennzeichnet, dass** zum Verbinden von Modulen gleicher oder unterschiedlicher Länge (L) ein Modulverbinder (17) vorgesehen ist, dessen Aussendurchmesser (AD) auf den Innendurchmesser (ID) der Einstecköffnung (21) abgestimmt ist, und dass zum Verbinden des Modulverbinders (17) mit dem Nagel (2) zusätzlich ein manschettenartiges Modul (3, 3.1-3.4) mit mindestens zwei in Achsrichtung (LA) des Moduls (3) geteilte, lösbar miteinander verbundene Halbschalen (8.1, 8.2) vorgesehen ist, die miteinander eine sich axial erstreckende Durchführung (9) zum Einführen des Nagelhalses (7) in das eine Ende und des Modulverbinders (17) in das andere Ende der Durchführung (9) ausbilden und die koaxial in Längsrichtung des Moduls (3, 3.1-3.4) angeordnete, jeweils senkrecht zur Achse (LA) positionierte, zueinander korrespondierende Ausnehmungen (12.1, 12.2) zur Aufnahme des Spannmittels (16) aufweisen, das im gespannten Zustand den Nagelhals (7) und den Modulverbinder (17) in der Durchführung (9) ausschließlich mittels Reibschluss spannbackenartig mit einem gegen axiale Verschiebung und Rotation ausreichenden Anzugsmoment klemmend fixiert.

4. Gelenkprothese nach Anspruch 1 oder 3, **dadurch gekennzeichnet, dass** das Modul (3, 3.1-3.4) im Wesentlichen eine zylindrische Form aufweist und die Halbschalen (8.1, 8.2) im Wesentlichen gleichartig ausgebildet sind.

5. Gelenkprothese nach einem der Ansprüche 1, 3 und 4, **dadurch gekennzeichnet, dass** das Modul(3,3.1-3.4) längenvariabel ist und Module mit verschiedenen Längen miteinander kombinierbar sind.

6. Gelenkprothese nach Anspruch 1, **dadurch gekennzeichnet, dass** zum Verbinden von Modulen (3, 3.1-3.4) gleicher oder unterschiedlicher Länge (L) ein Modulverbinder (17) vorgesehen ist, dessen Außendurchmesser (AD) auf den Innendurchmesser (ID) der Durchführung (9) oder der Einstecköffnung (21) abgestimmt ist.

7. Gelenkprothese nach Anspruch 1 oder 2, **dadurch gekennzeichnet, dass** die Durchführung (9) oder die Einstecköffnung (21) an ihren Innenwandungen (10, 27) eine quer zur Längsrichtung (LA) des Moduls (3, 3.1-3.4) bzw. des Aufnahmekopfes (20) angeordnete Profilierung (11, 28) zum Verhindern von Mikrobewegungen, vorzugsweise Fretting, zwischen Nagelhals (7) und Modulverbinders (17) im Modul (3,3.1-3.4) bzw. in der Einstecköffnung (21) aufweist.

8. Gelenkprothese nach Anspruch 3 oder 7, **dadurch gekennzeichnet, dass** der Modulverbinder (17) aus Vollmaterial besteht.

9. Gelenkprothese nach Anspruch 1 oder 3, **dadurch gekenzennzeichnet,** dass der Durchmesser der Module (3, 1.1-3.4) unterschiedlicher Länge (L) gleich ist.

10. Gelenkprothese nach Anspruch 1 oder 2, **dadurch gekennzeichnet, dass** die Spannmittel (16,26) aus einer Innensechskantschraube (15,30) und einem in der korrespondierenden Ausnehmung (12.1,12.2 bzw. 25.1, 25.2) vorgesehenen Innengewinde (14, 29) bestehen.

11. Gelenkprothese nach Anspruch 10, **dadurch gekennzeichnet, dass** das Spannmittel (16, 26) durch Aufbringen eines bestimmten Anzugsmoments gegen Lösen gesichert ist.

12. Gelenkprothese nach Anspruch 1 oder 2, **dadurch gekennzeichnet, dass** der Nagel (2) verschiedene Längen (L) aufweist, wobei der Durchmesser des Nagelhals der Nägel verschiedener Längen gleich ist.

13. Gelenkprothese nach einem der Ansprüche 1 bis 12, **dadurch gekennzeichnet, dass** das Halsstück (19) mit und ohne Modul (3, 3.1-3.4) mit dem Nagel (2) unterschiedlicher Länge kombinierbar ist.

14. Gelenkprothese nach Anspruch 1 bis 13, **dadurch gekennzeichnet, dass** alle Teile der Prothese aus einer biokompatiblen Legierung, vorzugsweise eine Titanlegierung, bestehen.

15. Gelenkprothese nach Anspruch 1 bis 13, **dadurch gekennzeichnet, dass** alle Teile der Prothese eine raue Oberfläche zur Förderung des Anwachsens des Knochens aufweist.

## Claims

1. A modular joint prosthesis with a curved head part, to the conical nose of which a push-on joint ball is fastened, and with a stem (2) insertable into the bone marrow canal of the long bone and having a stem neck (7), a connecting means being provided between head part (1) and stem neck (7) which holds the head part non-rotatably, **characterised in that** the connecting means comprises at least one collar-like module (3, 3.1-3.4) placed in the axial direction of the stem and having at least two half-shells (8.1, 8.2) divided in the axial direction (LA) of the module (3, 3.1-3.4) and joined undoably together, which half-shells together form an axially extending bushing (9) for insertion of the stem neck (7) into the bushing (9) and which comprise mutually corresponding openings (12.1, 12.2) arranged coaxially in the longitudinal direction of the module (3) and each positioned perpendicular to the axis (LA) for receiving a clamping means (16), which in the clamped state fixes the stem neck (7) in the bushing (9) solely by means of frictional engagement in the manner of a clamping jaw with a tightening torque sufficient to counter axial displacement and rotation.

2. A modular joint prosthesis with a shaft-shaped neck piece, to the conical nose of which a push-on joint ball is fastened, and with a stem (2) insertable into the bone marrow canal of the long bone and having a stem neck (7), a connecting means being provided between neck piece (19) and stem neck (7) which holds the neck piece (19) non-rotatably in a predetermined angular position, **characterised in that** the neck piece (19) is shaped at its distal end into a domed receiving head (20) capable of being pushed onto the stem neck (7) of the stem, which receiving head comprises an insertion opening (21) for insertion of the stem neck (7) or of a module connector (17), the head (20) being provided with a slot (23) which extends in the direction of the longitudinal axis (LA) of the neck piece (19) approximately as far as into the shaft (22), said slot being associated in each case with mutually corresponding openings (25.1, 25.2) positioned perpendicular to the axis (LA) for receiving a clamping means (26), which in the clamped state clampingly fixes the stem neck (7) or module connector (17) in the receiving head (20) of the neck piece (19) solely by means of frictional engagement known *per se* in the manner of a clamping jaw with a tightening torque sufficient to counter axial displacement and rotation.

3. A joint prosthesis according to claim 2, **characterised in that**, to connect modules of identical or different length (L), a module connector (17) is provided, the external diameter (AD) of which matches the internal diameter (ID) of the insertion opening (21), and **in that**, to connect the module connector (17) with the stem (2), a collar-like module (3, 3.1-3.4) is additionally provided with at least two half-shells (8.1, 8.2) divided in the axial direction (LA) of the module (3) and joined undoably together, which half-shells together form an axially extending bushing (9) for insertion of the stem neck (7) and of the module connector (17) respectively into the one end and into the other end of the bushing (9) and which comprise mutually corresponding openings (12.1, 12.2) arranged coaxially in the longitudinal direction of the module (3, 3.1-3.4) and each positioned perpendicular to the axis (LA) for receiving the clamping means (16), which in the clamped state clampingly fixes the stem neck (7) and the module connector (17) in the bushing (9) solely by means of frictional engagement in the manner of a clamping jaw with a tightening torque sufficient to counter axial displacement and rotation.

4. A joint prosthesis according to claim 1 or claim 3, **characterised in that** the module (3, 3.1-3.4) is substantially cylindrical in form and the half-shells (8.1, 8.2) are of identical construction.

5. A joint prosthesis according to one of claims 1, 3 and 4, **characterised in that** the module (3, 3.1-3.4) is variable in length, it being possible to combine together modules of different lengths.

6. A joint prosthesis according to claim 1, **characterised in that,** to connect modules (3, 3.1-3.4) of identical or different lengths (L), a module connector (17) is provided, the external diameter (AD) of which is matched to the internal diameter (ID) of the bushing (9) or of the insertion opening (21).

7. A joint prosthesis according to claim 1 or claim 2, **characterised in that** at its internal walls (10, 27) the bushing (9) or the insertion opening (21) comprises profiling (11, 28) arranged transversely of the longitudinal direction (LA) of the module (3, 3.1-3.4) or of the receiving head (20) respectively to prevent micro movements, preferably fretting, between stem neck (7) and module connector (17) in the module (3, 3.1-3.4) or in the insertion opening (21).

8. A joint prosthesis according to claim 3 or claim 7, **characterised in that** the module connector (17) consists of solid material.

9. A joint prosthesis according to claim 1 or claim 3, **characterised in that** the diameter of the modules (3; 1.1-3.4) of different lengths (L) is identical.

10. A joint prosthesis according to claim 1 or claim 2, **characterised in that** the clamping means (16, 26) consist of a hexagon socket screw (15, 30) and an internal thread (14, 29) provided in the corresponding opening (12.1, 12.2 or 25.1, 25.2 respectively).

11. A joint prosthesis according to claim 10, **characterised in that** the clamping means (16, 26) is secured against release by application of a given tightening torque.

12. A joint prosthesis according to claim 1 or claim 2, **characterised in that** the stem (2) has different lengths (L), the diameter of the stem neck of the stems of different lengths being identical.

13. A joint prosthesis according to one of claims 1 to 12, **characterised in that** the neck piece (19) with and without the module (3, 3.1-3.4) may be combined with the different length stem (2).

14. A joint prosthesis according to claim 1 to claim 13, **characterised in that** all the parts of the prosthesis consist of a biocompatible alloy, preferably a titanium alloy.

15. A joint prosthesis according to claim 1 to claim 13, **characterised in that** all the parts of the prosthesis have a rough surface for promoting growth of bone thereon.

## Revendications

1. Prothèse articulaire modulaire avec une partie de tête de forme courbe, sur l'embout conique de laquelle une rotule articulaire emboîtable est fixée, et avec un clou (2) pourvu d'un collet (7), apte à être inséré dans le canal médullaire de l'os long, un moyen d'assemblage étant prévu entre la partie de tête (1) et le collet (7) du clou, qui maintient la partie de tête avec blocage en rotation, **caractérisée en ce que** le moyen d'assemblage comprend au moins un module (3, 3.1-3.4) de type manchon, disposé en direction axiale du clou et comprenant au moins deux demi-coques (8.1, 8.2) séparées en direction axiale (LA) du module (3, 3.1-3.4) et reliées entre elles de façon détachable, qui forment ensemble un passage (9) s'étendant axialement, permettant d'introduire le collet (7) dans ledit passage (9), et qui présentent des évidements (12.1, 12.2) correspondant entre eux, disposés coaxialement en direction longitudinale du module (3) et positionnés chacun perpendiculairement à l'axe (LA), servant à recevoir un moyen de serrage (16) qui, lorsqu'il est serré, bloque le collet (7) dans le passage (9) exclusivement par engagement par friction à la manière de mâchoires de serrage, avec un couple de serrage suffisant pour empêcher tout déplacement axial et toute rotation.

2. Prothèse articulaire modulaire avec une pièce formant col en forme de tige, sur l'embout conique de laquelle une rotule articulaire emboîtable est fixée, et avec un clou (2) pourvu d'un collet (7), apte à être inséré dans le canal médullaire de l'os long, un moyen d'assemblage étant prévu entre la partie formant col (19) et le collet (7) du clou, qui maintient la partie formant col (19) dans une position angulaire prédéterminée avec blocage en rotation, **caractérisée en ce que** la pièce formant col (19) est conformée à son extrémité distale en une tête de réception (20) en forme de casque emboîtable sur le collet (7) du clou, qui présente une ouverture d'insertion (21) permettant d'introduire le collet (7) ou un raccord de module (17), la tête (20) étant pourvue d'une fente (23) s'étendant en direction de l'axe longitudinal (LA) de la pièce formant col (19) pratiquement jusque dans la tige (22) et à laquelle sont associés des évidements (25.1, 25.2) correspondant entre eux, positionnés chacun perpendiculairement à l'axe (LA), servant à recevoir un moyen de serrage (26) qui, lorsqu'il est serré, bloque le collet (7) ou le raccord de module (17) dans la tête de réception (20) de la pièce formant col (19) exclusivement par engagement par friction en soi connu à la manière de mâchoires de serrage, avec un couple de serrage suffisant pour empêcher tout déplacement axial et toute rotation.

3. Prothèse articulaire selon la revendication 2, **caractérisée en ce que,** pour assembler des modules de longueur (L) identique ou différente, un raccord de module (17) est prévu, dont le diamètre extérieur (AD) est adapté au diamètre intérieur (ID) de l'ouverture d'insertion (21), et **en ce que**, pour assembler le raccord de module (17) avec le clou (2), il est prévu, en plus, un module (3, 3.1-3.4) de type manchon, comprenant au moins deux demi-coques (8.1, 8.2) séparées en direction axiale (LA) du module (3) et reliées entre elles de façon détachable, qui forment ensemble un passage (9) s'étendant axialement, permettant d'introduire le collet (7) dans l'une et le raccord de module (17) dans l'autre extrémité dudit passage (9) et qui présentent des évidements (12.1, 12.2) correspondant entre eux, disposés coaxialement en direction longitudinale du module (3, 3.1-3.4) et positionnés chacun perpendiculairement à l'axe (LA), servant à recevoir le moyen de serrage (16) qui, lorsqu'il est serré, bloque le collet (7) et le raccord de module (17) dans le passage (9) exclusivement par engagement par friction à la manière de mâchoires de serrage, avec un couple de serrage suffisant pour empêcher tout déplacement axial et toute rotation.

4. Prothèse articulaire selon la revendication 1 ou 3, **caractérisée en ce que** le module (3, 3.1-3.4) présente une forme sensiblement cylindrique et les demi-coques (8.1, 8.2) sont réalisées de manière sensiblement identique.

5. Prothèse articulaire selon l'une des revendications 1, 3 et 4, **caractérisée en ce que** le module (3, 3.1-3.4) est de longueur variable et les modules de longueurs différentes peuvent être combinés entre eux.

6. Prothèse articulaire selon la revendication 1, **caractérisée en ce que,** pour assembler des modules (3, 3.1-3.4) de longueur (L) identique ou différente, un raccord de module (17) est prévu, dont le diamètre extérieur (AD) est adapté au diamètre intérieur (ID) du passage (9) ou de l'ouverture d'insertion (21).

7. Prothèse articulaire selon la revendication 1 ou 2, **caractérisée en ce que** le passage (9) ou l'ouverture d'insertion (21) présente sur ses parois intérieures (10, 27) un profilage (11, 28) placé transversalement à la direction longitudinale (LA) du module (3, 3.1-3.4) resp. de la tête de réception (20), servant à empêcher des microdéplacements, de préférence le fretting, entre le collet (7) du clou et le raccord de module (17) dans le module (3,3.1-3.4) resp. dans l'ouverture d'insertion (21).

8. Prothèse articulaire selon la revendication 3 ou 7, **caractérisée en ce que** le raccord de module (17) est en matériau plein.

9. Prothèse articulaire selon la revendication 1 ou 3, **caractérisée en ce que** le diamètre des modules (3, 1.1-3.4) de longueur (L) différente est le même.

10. Prothèse articulaire selon la revendication 1 ou 2, **caractérisée en ce que** les moyens de serrage (16, 26) sont constitués d'une vis à six pans creux (15, 30) et d'un filetage intérieur (14, 29) ménagé dans l'évidement (12.1, 12.2 resp. 25.1, 25.2) correspondant.

11. Prothèse articulaire selon la revendication 10, **caractérisée en ce que** le moyen de serrage (16, 26) est protégé contre un desserrage par application d'un couple de serrage déterminé.

12. Prothèse articulaire selon la revendication 1 ou 2, **caractérisée en ce que** le clou (2) présente différentes longueurs (L), le diamètre du collet des clous de longueurs différentes étant le même.

13. Prothèse articulaire selon l'une des revendications 1 à 12, **caractérisée en ce que** la pièce formant col (19) peut être combinée avec ou sans module (3, 3.1-3.4) avec le clou (2) de longueur différente.

14. Prothèse articulaire selon la revendication 1 à 13, **caractérisée en ce que** toutes les pièces de la prothèse sont constituées d'un alliage biocompatible, de préférence d'un alliage de titane.

15. Prothèse articulaire selon la revendication 1 à 13, **caractérisée en ce que** toutes les pièces de la prothèse présentent une surface rugueuse de manière à favoriser la repousse osseuse.
